# EUROPEAN PATENT APPLICATION

(11) **EP 4 390 963 A1**
(43) Date of publication of application: **26.06.2024**
(21) Application number: 22215830.5
(22) Date of filing: 22.12.2022
(51) Int. Cl.: G16H 40/40, A61B 6/00, H05G 1/00

(54) **METHOD FOR DETERMINING A WORKFLOW PHASE OF A MEDICAL SYSTEM IN A MEDICAL ENVIRONMENT**

(71) Applicant: Koninklijke Philips N.V., 5656 AG Eindhoven (NL)
(72) Inventor: IMMINK, Albert Hendrik Jan, Eindhoven (NL); AGAFONOV, Aleksei, Eindhoven (NL); BASTIAANSEN, Thomas, 5656AG Eindhoven (NL)
(74) Representative: Philips Intellectual Property & Standards

(57) **Abstract**

A method for determining a workflow phase of a medical system (201) in a medical environment, comprising: obtaining, by a processor, signals (300, 400, 500) from at least one sensor (202, 203, 204, 205), wherein the at least one sensor (202, 203, 204, 205) is external to the medical system (201) and configured to monitor the medical system (201) (S100); providing, by the processor, a workflow phase determining algorithm (401, 501) configured to determine workflow phase data of the medical system (201) (S200); determining, by the processor, the workflow phase data by utilizing the workflow phase determining algorithm (401, 501) and the obtained signals (300, 400, 500) (S300).

## Description

### FIELD OF THE INVENTION

The invention relates to a method for determining a workflow phase of a medical system in a medical environment, to a device for determining a workflow phase of a medical system in a medical environment, to a system and to a computer program.

### BACKGROUND OF THE INVENTION

Medical procedures of medical systems such as diagnostics in a MRI system are well known in the state of the art. These medical procedures comprise in general different workflow phases. It is crucial to know at what workflow phase such a medical procedure is.

It has now become apparent that there is a need to provide a method for determining a workflow phase of medical system.

### SUMMARY OF THE INVENTION

In view of the above, it is an object of the present invention to provide a method that allows improving the determining of a workflow phase of a medical system in a medical environment.

These and other objects, which become apparent upon reading the following description, are solved by the subject matter of the independent claims. The invention provides a method, a device, a system and computer program. The dependent claims refer to preferred embodiments of the invention.

In one aspect of the present disclosure a method for determining a workflow phase of a medical system in a medical environment is provided, comprising:
obtaining, by a processor, signals from at least one sensor, wherein the at least one sensor is external to the medical system and configured to monitor the medical system;
providing, by the processor, a workflow phase determining algorithm configured to determine workflow phase data of the medical system;
determining, by the processor, the workflow phase data by utilizing the workflow phase determining algorithm and the obtained signals.

The term workflow phase, as used herein, is to be understood broadly and may refer to any step or state in a procedure carried out by a medical system. The workflow phase may comprise a preparation phase, start up phase, an operation phase, a waiting phase, a follow up phase, a boot up phase of a part of the medical system, a positioning phase of a part of the medical system or a subject to be treated by the medical system. The workflow phase may be described by workflow phase data.

The term workflow phase data, as used herein, is to be understood broadly and may relate to any data configured to describe the workflow phase. The workflow phase data may comprise a temporal duration of a workflow phase, a time of a workflow phase, a name of a workflow phase (e.g. start up phase), a medical system state (e.g. medical system off, medical system on hold). The workflow phase data may comprise the workflow phase.

The term medical system, as used herein, is to be understood broadly and may relate to any system used in medical environment. The medical system may comprise one or more medical units that are used in combination (e.g. a MRI system and patient table). The medical system may comprise one or more subunits (e.g. positioning unit of a MRI system, a coil of a MRI system). The medical system may comprise a MRI system, an X-ray system and/or a CT system.

The term medical environment, as used herein, is to be understood broadly and may relate to any environment in which a medical system is. The medical environment may be a treatment room in a hospital or in a medical practice.

The term signal, as used herein, is to be understood broadly and may relate to any data provided from a sensor. The signal may comprise analogue or digital data. The signal may comprise raw data or at least partially processed data. The signal may be for example a noise signal, a temperature signal, a vibration signal, an acoustic signal and/or an infrared signal. The signal may comprise different signals from one or more sensors. The signals may be obtained by the processor by means of transmitting means (e.g. a wireless data interface, a bus system between the processor and the sensor).

The term sensor, as used herein, is to be understood broadly and may relate to any measuring device configured to measure a physical value. The sensor may be a vibration sensor, an acoustic emission sensor, a temperature sensor and/or a noise sensor. The sensor may be a single entity or distributed over several entities. The sensor may base on one or more physical measurement principles. The sensor may comprise one or more sensors (e.g. a sensor array, or a temperature sensor and a vibration sensor).

The term external, as used herein, is to be understood broadly and means that the sensor is not part of the medical system. In other words, the sensor is a separate unit in relation to the medical system. The sensor may be configured to operate independently of the medical system. The sensor (e.g. vibration sensor) may be placed on a housing of the medical system to monitor the medical system.

The term workflow phase determining algorithm, as used herein, is to be understood broadly and may relate to any logic configured to determine a workflow phase of a medical system. The workflow phase determining algorithm may be an analytical algorithm and/or a heuristic algorithm. The workflow phase determining algorithm may comprise a regression model or an AI model (e.g. machine learning model). The workflow phase determining algorithm may comprise a classification algorithm.

In other words, the basic idea of the invention may comprise analysing signals from one or more sensors placed in a vicinity of the medical system in order to determine a workflow phase of a medical system. The method may be a computer-implemented method carried out by a processor. This may be advantageous, as it helps to determine at what stage the medical procedure carried out by the medical system is right now. The information about the workflow phase may be used to analyse an efficiency of the medical system and/or the medical procedure. This may be advantageous in order to identify bottlenecks and/or failures. This may be advantageous in order to trigger accompanying processes or analysis. This may be advantageous in terms of quality control. This may be advantageous in terms of reliability, as a method that is independent (due to an external sensor and/or an external processor) from the medical system is used.

Due to ageing populations worldwide, scarcity of clinical personnel and pressure on healthcare budgets there may be a strong demand to increase an efficiency of clinical operations and to improve an experience of clinical staff on a day-to-day basis. Regularly, these demands may be expressed in a so-called quadruple aim: improved patient experience, better health outcomes, improved staff experience and lower cost of care.

The following problems may be addressed by the proposed invention: Many different modalities may have to be connected and equipped with additional sensors to get an overarching overview of all the relevant processes in a care environment (e.g., a catheter lab or a similar hospital setting). The design of sensors may be generally a costly and lengthy trajectory (involving formal verification, validation, and regulatory release) and a large installed base needs to be built up before widely available benefits can be reaped from such a system. Ideally, a vendor agnostic method may be applied that can potentially sense a wide range of equipment that is used in the clinical procedure, including third party devices.

A further problem overcome by the invention may be that without this invention all failure detection and prediction algorithms would need to run in parallel in an always-on mode. That may have some severe technical disadvantages: An edge device with a high computational power may be needed to support the algorithm executions. This may consume high power and likely leads to a costly device. The algorithms may continuously process sound/vibration/RF signals, even when this is not relevant. This may lead to false positives or spurious warnings. Some algorithms may drift or get stuck in local loops if ran for longer period with unintended inputs or noisy inputs (e.g., background sounds, or sounds from other elements/parts of the medical system).

For this reason, it would be advantageous to smartly select the right algorithm at the right moment, i.e. during those periods of time the estimate can best be done (e.g. for gantry angle detection) or when the failure can occur (e.g. arcing can only occur when X-ray is generated, and the high-voltage system is active).

Another additional benefit may be that the invention allows triggering of targeted anomaly detectors that can selectively trigger the discarding of data or sending of data to the cloud. Anomaly detection may be much easier when it only needs to focus on specific workflow phases or subunits of the medical system of which the normal behaviour is known.

To achieve the above goals, there may be a need to monitor workflow phases to populate dashboards and to identify inefficiencies (slow/delayed procedures, waiting times, repeat diagnostic procedures, equipment downtime, etc.) and to reduce an administrative burden of a healthcare staff. The latter can e.g. be done by collecting data from the different modalities used in the clinical procedure and to identify key information (e.g. using artificial intelligent algorithms) and place this on a workflow phase timeline. Vice versa, also key information can be identified by keeping track of important phase transitions in this workflow.

Another reason to classify the workflow into different phases may be that in each phase specific elements of the system are active and produce characteristic sounds, RF fields and/or other measurable signals. Generally, a failure or deterioration of a subunit of a medical system may become detectable only in these active phases. This may be advantageous to execute different targeted monitoring and failure prediction algorithms consecutively, each in the workflow phase that may be relevant for that failing element.

This may mean that for all demands (dash boarding, administration automation and targeted prediction algorithms) the ability to keep track of the (patient, staff, and equipment) workflow may be a key enabler.

The invention may propose to equip staff and the environment with one or more sensors (e.g. wearing RF-ID badges, position sensors for locating mobile equipment).
The invention may focus on the enabling of different monitoring and failure predicting algorithms, each at the right moment in time.

The invention proposes a method that allows patient/procedure workflow phase detection by processing signals from one or more sensors that may be sensitive in all directions (i.e. omni-sensitive). The sensors can be arranged next to or onto modalities that are used in a healthcare procedure. The result can be applied for dash boarding, administration automation or the triggering of targeted algorithms used for predictive maintenance or failure monitoring.

The invention may generally be used in a hospital setting at locations where patients are diagnosed or treated using multiple modalities and devices. A proposed system can for example include a computer tomography (CT) system, an electrically adjustable patient bed, a C-arm X-ray system, magnetic resonance (MR) systems, ultrasound systems, catheter systems, respiratory systems (like ventilators). Some of these systems may have fixed locations in the room. Some of these systems may be flexible to move around or even be implemented on mobile carts (depending on the procedure or the phase of the procedure).

In such an environment, a plurality of sensors (e.g. of different types) can be positioned at appropriate locations. The sensors can be arranged next to or onto an equipment (e.g. to sense vibrations) or they can be arranged at a certain distance from the equipment (e.g. to detect sounds or RF electromagnetic emissions coming from the equipment) or a combination thereof.

An interesting and important observation might be that a very significant part of the signal content is outside the audible spectrum and can only be recorded with wide-band microphones using high sampling frequencies. The sensor used in the proposed method may therefore be a wide-band microphone using a high sampling frequency.

In the area CT systems for example the following workflow phases can be determined: Collimator adjustment, gantry rotation (speed-up, steady-state rotation, coast down) and X-ray generation.

The present invention may allow for uniquely identifying workflow-related operations, e.g.: movement of the patient table in different directions and angles, detection of air-fans switching on and off and/or regulated at different speeds.

In the area of C-arm X-ray systems, the following workflow phases may be determined: movement of the C-arm along the ceiling rail, movement of the C-arm around the patient in different orientations, movement of the boom-monitor. This may apply for both fixed and mobile C-arm systems.

In the area of CT systems, the following workflow phases may be determined: movement of the CT-system over the ground rail in case of CT on rails configurations.

In the area of MR systems, the following workflow phases may be determined: detection of magnetic fields (in particular magnetic field modulations-gradients) in MR systems and/or detection of RF activation pulses.

In the area of DXR systems, the following workflow phases may be determined: movement of an XRT and/or a detector along a ceiling suspension, e.g. a rail system.

According to an embodiment, the method may comprise: using, by the processor, the workflow phase data to select at least one evaluation algorithm for a medical system corresponding to a workflow phase comprised in the workflow phase data; and/or
providing, by the processor, the determined workflow phase data for further processing, wherein the further processing comprises displaying the workflow phase data and/or analysing the workflow phase data.

The term evaluation algorithm, as used herein, is to be understood broadly and may relate to any algorithm configured to analyse a workflow phase. The evaluation algorithm may relate to an algorithm configured to analyse a subunit of the medical system (e.g. a coil of the MRI system or a patient table). The evaluation algorithm may detect a failure of at least a part of the medical system (e.g. start-up noise of a coil of a MRI system). The evaluation algorithm may localise a failure in at least a part of the medical system (e.g. uncommon vibration in a drive of a C-arm of a projection X-ray imaging system). The method may provide a plurality of evaluation algorithms.

The proposed method for determining a workflow phase of a medical system in a medical environment may comprise selecting the one or more evaluation algorithms in dependency of the determined workflow phase. For example, when a workflow phase "start-up MRI coil is determined", the evaluation algorithm "quality control coil of the MRI system" is selected and executed.

The proposed method for determining a workflow phase of a medical system in a medical environment may comprise providing a selection table comprising the one or more evaluation algorithms. Based on the determined workflow phase one or more evaluation algorithms may be selected from the selection table.

The evaluation algorithm may be executed on a separate entity (e.g. a cloud). The processor that carries out the method described above may not have to be as powerful to execute the evaluation algorithm in case it only selects the evaluation algorithm. The processor may further only provide a trigger signal for another entity to execute the selected evaluation algorithm. This may be advantageous in terms of resource efficiency of the processor.

This may be advantageous in terms of quality control, reliability, efficiency and/or availability of the medical system. This may further be advantageous, as the evaluation algorithm is only executed when it is needed. This may save resources and may lead to better results (due to less noise signals from other workflow phases).

The displaying of the determined workflow phase data may comprise presenting the determined workflow phase on a screen, a dashboard, a tablet and/or a mobile phone. This may be advantageous in terms of user friendliness, reduction of complexity and/or real time analysis capability.

The analysing of the workflow phase data may relate to an administration analysis of the workflow phase (e.g. comparison of determined workflow phase duration with reference values). This may be advantageous in terms of an efficiency increase. The term administration analysis, as used herein, is to be understood broadly and may relate to any process analysis of a workflow phase. The administration analysis may comprise a determining of one or more key performance indicators such as for example system availability and/or productivity.

The workflow phase determining can be used to build up a timeline as a basis for post-procedure reporting. In addition, automated administration (e.g. key-frame extraction from fluoroscopy streams) can benefit from the workflow phase data. The term administration may comprise administrative tasks (e.g. extraction of CT image for documentation).

According to an embodiment, the at least one evaluation algorithm may comprise at least one of the following: a failure detection algorithm of at least a part of the medical system, a position estimation algorithm of at least a part of the medical system, a failure localization algorithm of at least a part of the medical system and a failure prediction algorithm of at least a part of the medical system.

The term failure detection algorithm, as used herein, is to be understood broadly and may relate to any algorithm configured to detect a failure in a workflow phase and/or a corresponding part of the medical system that is active during this workflow phase (e.g. a coil of the MRI system). The failure detection algorithm may comprise an anomaly detection. This may be advantageous as it allows a targeted monitoring of a subunit.

The term position estimation algorithm, as used herein, is to be understood broadly and may relate to any algorithm that allows determining a position of a movable part of a medical system (e.g. C-arm of a projection X-ray imaging system). The position estimation algorithm may comprise a multi body model. Based, for example, on a noise signal that is indicative of position of a part of the medical system, a position of the part of the medical system may be determined. This may be advantageous in terms of quality control, as it reveals a current position of the part of the medical system. The information may be used for further analysis or may be used to readapt the positon of the part of the medical system.

The term failure localization algorithm, as used herein, is to be understood broadly and may relate to any algorithm configured to identify a failure of a part of the medical system. The failure may relate to at least a part of the medical system or even to a specific area of a part of the medical system. This may be advantageous in terms of quality control.

The term failure prediction algorithm, as used herein, is to be understood broadly and may relate to any algorithm configured to predict a failure based on the obtained signals and/or further information/data relating to the medical system. The failure prediction algorithm may be based on an anomaly analysis. This may be advantageous in terms of quality control.

In an embodiment, the method may comprise executing the at least one selected evaluation algorithm, and in particular presenting a result of the executed at least one selected evaluation algorithm on a user interface. The method may execute the selected evaluation algorithm on the processor. Alternatively, the method may execute the selected evaluation algorithm on a separate entity, e.g. on a workstation or in a cloud. This may be advantageous as it allows using a processor with low performance requirements, as the power demanding evaluation algorithm is executed on a separate powerful entity.

The method may execute targeted monitoring and/or targeted failure prediction algorithms at the relevant moment in time, i.e. during the relevant workflow phases of the procedure. For this purpose, the method may use the workflow phase determining algorithm to enable and/or trigger a set of evaluation algorithms that focus on a specific failure and/or a specific element of the system, e.g. one that focuses on failures in the collimator, one that does arcing detection and localisation, and/or one that predicts or localises other mechanical (wear-induced) failures.

The benefit of the selecting and executing the right evaluation algorithm may be that the different evaluation algorithms are only active when needed. Hence, these evaluation algorithms may not consume unneeded compute power or energy, or create unnecessary false positives or spurious warnings.

In an embodiment, the method may comprise the processor analysing the determined workflow phase data based on corresponding reference workflow phase data to obtain an analysing result, the analysing result particularly comprising identification of an anomaly in the workflow phase data. The method may comprise the processor transmitting the obtained signals from the at least one sensor to an analysing platform, in particular a platform on a cloud system, via a data connection for further analysis based on the analysing result or deleting the obtained signals based on the analysing result.

The term anomaly, as used herein, is to be understood broadly and may relate to any identified deviation in the determined workflow phase data from a reference value in the corresponding reference workflow phase data.

The term analysing platform, as used herein, is to be understood broadly and may relate to any separate entity in relation to the processor. The separate entity may be configured to execute one or more analysing algorithms. The analysing platform may be a cloud application or embedded in a workstation.

In other words, the method may analyse the obtained workflow phase data by comparing it to reference data for the corresponding determined workflow phase. In case an anomaly is detected in the workflow phase data, the raw data obtained from the sensor is sent for further analysing to the analysing platform otherwise the raw data is deleted. This may be advantageous, as unimportant data does not have to be stored or analysed. This may advantageously save resources.

In an embodiment, the at least one selected algorithm may be executed on a cloud system, wherein the processor and the cloud system are connected by a data connection, and wherein in particular the processor is implemented in an edge device. The data connection may comprise a bus system or a wireless data interface. This may be advantageous in terms of resource efficiency as the processor carrying out the method may be configured with low performance compared to the cloud system. The processor may be part of an edge device, such as a tablet or smart phone. The edge device may be a device on the medical system premises.

In an embodiment, the method may comprise signal processing of the obtained signals, wherein signal processing comprises signal conditioning, digitization, transformation and in particular feature extraction. This may be advantageous in terms of data quality, reliability and quality control.

Prior to the workflow phase determining algorithm is executed, some signal processing is carried out. The signal processing may comprise conditioning and/or digitization and/or feature extraction. Feature extraction may e.g. be done by spectral analysis using wavelet transformation or Short-Time Fourier Transform (STFT). Feature extraction may comprise using one or more bandpass filters in combination with a power detector to extract unique spectral fingerprints (i.e. feature) of at least a part of the medical system in a certain workflow phase.

In an embodiment, the workflow phase determining algorithm may be based on a machine learning algorithm trained to predict the workflow phase data, in particular the workflow phase comprised in the workflow phase data. The machine learning model preferably may comprise decision trees, naive bayes classifications, nearest neighbours, neural networks, convolutional neural networks, generative adversarial networks, support vector machines, linear regression, logistic regression, random forest and/or gradient boosting algorithms.

In an embodiment, the workflow phase determining algorithm may be based on a classification algorithm, in particular a classification algorithm configured to determine the workflow phase based on the obtained signals.

In an embodiment, the at least one sensor may comprise at least one of the following: an acoustic sensor, a vibration sensor, an electromagnetic sensor and a thermal sensor. The at least one sensor may be placed for intended use in a vicinity of the medical system or on a surface of the medical system or at least a part of the medical system.

In an embodiment, the medical system may comprise one or more parts and the medical system may comprise at least one of the following: a CT system, an X-Ray system, a MR system, an ultrasound system, a catheter system, a respiratory system and a patient table.

In an embodiment, the obtained signal may stem from a source configured to generate a synthetic signal and the source may be positioned on the medical system. The synthetic signal may be, for example, a noise signal or a heat signal. By comparing the obtained signal with a corresponding reference signal a position of at least a part of the medical system may advantageously be determined. For example, the sensor may thus capture the acoustic source originating from the spinning gantry.

A further aspect relates to a device for determining a workflow phase of a medical system in a medical environment, the device comprising means for carrying out the steps of the method described above. The means may comprise a processor, a computing unit, desktop pc, a workstation. The means may be a single entity or distributed on several entities. The means may comprise one or more interfaces for communicating with one or more sensors and/or other entities (e.g. cloud, analysing platform) and/or a user.

A further aspect relates to a system comprising a device described above and a medical system, at least one sensor and optionally a user interface. The medical system may be a MRI system, a CT system or the like. The system may comprise a screen or dashboard for presenting the determined workflow phase and/or analysing results of the one or more evaluation algorithms.

Another aspect relates to a computer program comprising instructions which, when the program is executed by a computer, cause the computer to carry out the method described above. Another aspect relates to a computer-readable medium comprising instructions which, when executed by a computer, cause the computer to carry out the method described above.

The computer program might be stored on a computer unit, which might also be part of an embodiment. This computer unit may be configured to perform or induce performing of the steps of the method described above. Moreover, it may be configured to operate the components of the above-described device. The computing unit can be configured to operate automatically and/or to execute the orders of a user. A computer program may be loaded into a working memory of a data processor. The data processor may thus be equipped to carry out the method according to one of the preceding embodiments. This exemplary embodiment of the invention covers both, a computer program that right from the beginning uses the invention and computer program that by means of an update turns an existing program into a program that uses invention. Further, on, the computer program might be able to provide all necessary steps to fulfil the procedure of an exemplary embodiment of the method as described above. According to a further exemplary embodiment of the present invention, a computer readable medium, such as a CD-ROM, USB stick or the like, is presented wherein the computer readable medium has a computer program stored on it which computer program is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present invention, a medium for making a computer program available for downloading is provided, which computer program is arranged to perform a method according to one of the previously described embodiments of the invention.

It is noted that the above embodiments may be combined with each other irrespective of the aspect involved. Accordingly, the method may be combined with structural features of the device and/or system of the other aspects and, likewise, the device and the system may be combined with features of each other, and may also be combined with features described above with regard to the method.

These and other aspects of the present invention will become apparent from and elucidated with reference to the embodiments described hereinafter.

### BRIEF DESCRIPTION OF THE DRAWINGS

Exemplary embodiments of the invention will be described making reference to the following drawings.
Fig. 1 is a schematic view of the method for determining a workflow phase of a medical system in a medical environment;
Fig. 2 shows schematically a device for determining a workflow phase of a medical system in a medical environment;
Fig. 3 shows schematically a system for determining a workflow phase of a medical system in a medical environment;
Fig. 4 shows exemplary signal analysing results;
Fig. 5 shows schematically an example for selecting an evaluation algorithm; and
Fig. 6 shows schematically an example for anomaly detection.

### DETAILED DESCRIPTION OF EMBODIMENTS

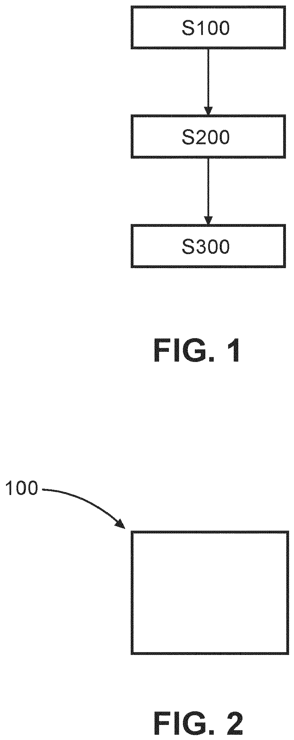

Fig. 1 is a schematic view of the method for determining a workflow phase of a medical system in a medical environment according to the present disclosure.

Step 100 comprises obtaining, by a processor, signals from at least one sensor, wherein the at least one sensor is external to the medical system and configured to monitor the medical system. The processor is, in the present example, part of an edge device (e.g. a tablet) that is used in the medical environment of the medical system. The medical system, is in the present example, a CT system. The at least one external sensor is, in the present example, an acoustic sensor arranged apart from the CT system. The acoustic sensor is configured to measure acoustic signals (i.e. here sound). The acoustic sensor is connected (by means of a wireless interface) with the edge device in order to transmit signals from acoustic sensor.

Step 200 comprises providing, by the processor, a workflow phase determining algorithm configured to determine workflow phase data of the medical system (S200).

The workflow phase determining algorithm is, in the present example, a classification algorithm that is based on an AI algorithm trained to determine a workflow phase of a medical system. The AI algorithm may be trained by existing data from the field or from test setups. Alternatively, the workflow phase determining algorithm may be an analytical algorithm.

In the present example, the obtained signals are, for example, additionally processed. The signal processing of the obtained signals may comprise signal conditioning, digitization, transformation and in particular feature extraction. The optional signal processing is carried out before step S300. The signal processing is carried out, in the present example, by the processor. The feature extraction can e.g. be done by a spectral analysis using wavelet transformation or Short-Time Fourier Transform (STFT).

Step 300 comprises determining, by the processor, the workflow phase data by utilizing the workflow phase determining algorithm and the obtained signals. The workflow phase data may comprise, in the present example, one of the following: a temporal duration, a time, an assignment to a workflow phase, a workflow state. In the present example, the workflow phase "Gantry of the CT system is moving" is determined.

In addition, the method may comprise: using, by the processor, the workflow phase data to select at least one evaluation algorithm for a medical system corresponding to a workflow phase comprised in the workflow phase data; and/or providing, by the processor, the determined workflow phase data for further processing, wherein the further processing comprises displaying the workflow phase data and/or analysing the workflow phase data.

In the present example, the method selects, from the plurality of evaluation algorithms, the position estimation algorithm for the Gantry of the CT system. The obtained sensor signals may be used by the position estimation algorithm to determine a position (i.e. Gantry angle) of the Gantry of the CT system.

In addition, other evaluation algorithms may be selected, such as a failure prediction algorithm for a collimator of the CT system or a failure detection algorithm for mechanical wear.

The method may further execute the selected evaluation algorithms. The execution may be carried out, in the present example, in a cloud application. Hence, the method triggers the execution in the cloud. The processor and the cloud system may be connected by a data connection. The execution may alternatively be carried out by the processor.

The determined workflow phase data may be presented on the edge device, a screen and/or a dashboard to a user. The results from the evaluation algorithms may also be presented to the user by the above mentioned means and/or an user interface (e.g. the edge device).

In addition, the method may further comprise analysing the determined workflow phase data based on corresponding reference workflow phase data to obtain an analysing result. The analysing result may particularly comprise identification of an anomaly in the workflow phase data. The processor may transmit the obtained signals from the at least one sensor to an analysing platform, in particular a platform on a cloud system, via a data connection for further analysis based on analysing result. Alternatively, the processor may delete the obtained signals based on the analysing result. In the present example, no anomaly has been detected in the workflow phase "Gantry of the CT system is moving" and therefore the obtained signals have been accordingly deleted.

Fig. 2 shows a device 100 for determining a workflow phase of a medical system in a medical environment.

The device 100 comprises means for carrying out the steps of the method described above. The means comprise, in the present example, a processor of an edge device. The means comprise, in the present example, one interface to communicate with the acoustic sensors and at least one interface to communicate with other entities (e.g. cloud, analysing platform) and a user.

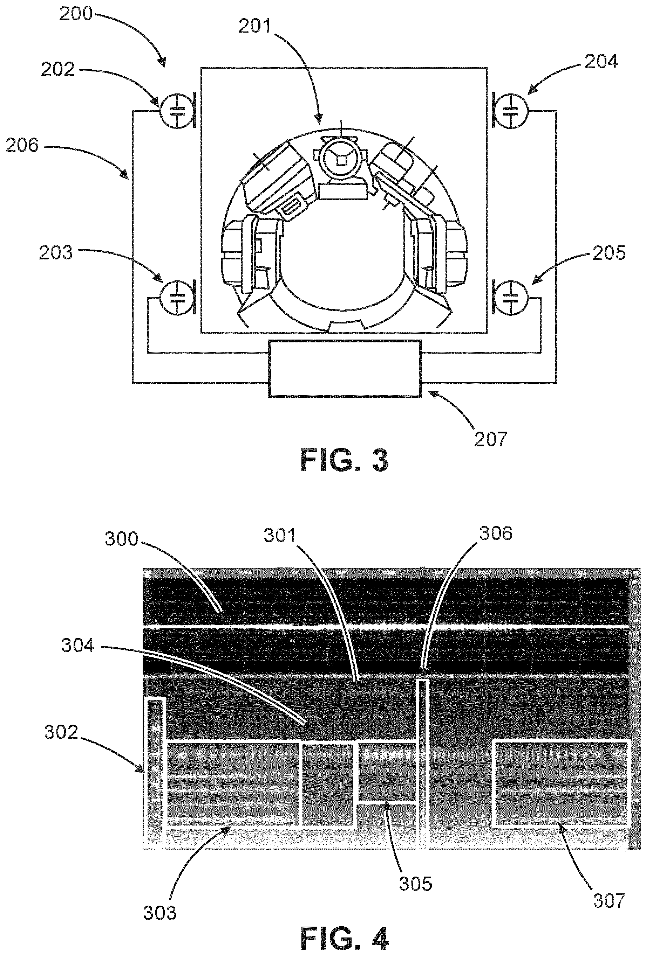

Fig. 3 shows a system 200 for determining a workflow phase of a medical system in a medical environment.

The system 200 comprises a device 207, for example a device as described above in the context of device 100. The medical environment may be a hospital. The system 200 comprises a medical system 201. The medical system 201 is, in the present example, a CT system. The system 200 comprises four acoustic sensors 202 to 205 that are linked to the device 207 described above by means of a wired connection 206. The system 200 may optionally further comprise a user interface (not shown) and/or a screen or dashboard (not shown) for presenting the determined workflow phase and/or the results of the one or more evaluation algorithms. It is noted that the number of acoustic sensors is merely exemplary and more or fewer sensors may be provided.

Fig. 4 shows exemplary signal analysing results.

Fig. 4 shows a signal waveform 300 of an acoustic signal of a sensor in time series and a corresponding spectrogram 301. The signals stem from a sensor monitoring a CT system. In the spectrogram, different work flow phases of the CT system can be identified. Section 302 of the spectrogram relates to a start up phase comprising collimator adjustment. Section 303 of the spectrogram relates to a speedup rotation phase of the CT system. Section 304 of the spectrogram relates to a steady rotation phase of the CT system. Section 305 of the spectrogram relates to an X-ray emission phase of the spectrogram. Section 306 of the spectrogram relates to an arc phase of the CT system. Section 307 relates to a gantry coast down phase of the CT system.

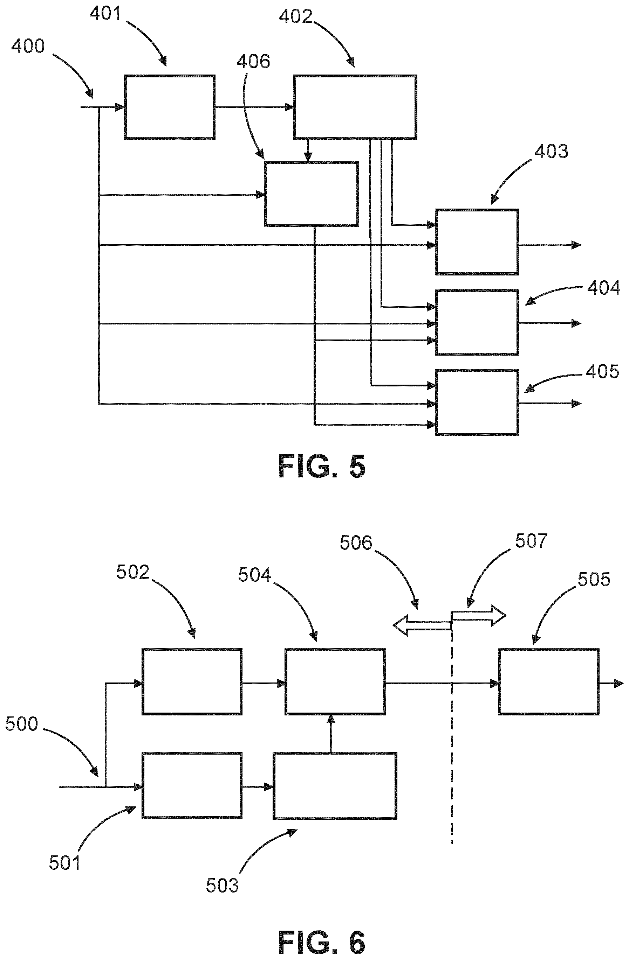

Fig. 5 shows schematically an example for selecting an evaluation algorithm according to the present disclosure.

The method obtains signals 400 from a sensor. The workflow phase determining algorithm 401 determines based on the obtained signals a workflow phase. The method then selects based on the determined workflow phase, by means of a selecting algorithm 402, one or more evaluation algorithms 403, 404 and/or 405. Evaluation algorithm 403 may comprise a failure detection/prediction algorithm for collimators. Evaluation algorithm 404 may comprise a failure detection/prediction algorithm for arcing detection. Evaluation algorithm 405 may comprise a failure detection/prediction algorithm for mechanical wear. The selection algorithm 402 may trigger an execution of the one or more evaluation algorithms. The selection algorithm 402 may also select a position estimation algorithm 406. The position estimation algorithm 406 may relate to a gantry estimation algorithm that provides as a result an estimated gantry angle. The positon estimation algorithm 406 may receive as input the obtained sensor signals 400. The position estimation algorithm 406 may provide for example the estimated gantry angle to the evaluation algorithms 404 and 405 as input. The evaluation algorithms 403 to 405 may provide the one or more evaluation results for further processing or for displaying to a user.

The position estimation algorithm may comprise, for example when used for estimation of a gantry angle, a pre-processing, a sound source detection and a controller loop. The pre-processing may comprise filtering raw data obtained by the sensor to only capture the relevant frequency of the sound source that the sensor is trying to track. The sound source detection may comprise detecting when the source is moving past a certain pre-determined point of the gantry rotational path. This can be accomplished by either using amplitude modulation or frequency modulation. Amplitude modulation may relate to the aspect that an amplitude is the highest when the source passes to the sensor (e.g. microphone). Frequency modulation may relate to the following aspect: Due to the velocity difference between the sensor and the rotating sound source, the observed signal's frequency may be shifted due to a doppler effect. A possible detection algorithm could observe the doppler effect with frequency modulation and map that to a gantry orientation. The controller loop may relate the following aspect: The detection of the sound source might introduce an error in the orientation estimation. To remove this error, for instance an oscillator in a control loop could be used. A possible implementation of the controller loop could be the digital phased lock loop (DPLL). The output of the controller loop would be the angle corresponding to the orientation of the gantry angle. When a separate system then detects that a failure has occurred, the gantry orientation angle can be read out. To provide user feedback, a small interface window (a physical interface on the edge device or a digital interface on the cloud) could show the continuously tracked gantry orientation angle during operation.

Fig. 6 shows schematically an example for anomaly detection according to the present disclosure.

The method comprises obtaining signals 500 from a sensor. The workflow phase determining algorithm 501 determines, based on the obtained signals, a workflow phase. Raw data of the signals 500 may be buffered by buffering unit 502 (i.e. local storage of the processor). An evaluation algorithm 503 may determine, based on the determined workflow phase and the obtained signals, an anomaly by comparing the obtained signals with reference signals of the corresponding workflow phase.

The method may comprise, by means of a processor (not shown), transmitting, via a data connection 504, for further analysis the raw data of the signals 500 to an analysing platform 505 (e.g. a cloud) for detailed analysis in case, an anomaly is detected. The method may comprise, by means of the processor, deleting the raw data of the signals 500 in case no anomaly is detected. The left side 506 in Fig. 6 represents a local side (i.e. edge side) and the rights side 507 represents an external side relating to a cloud.

The workflow phase determining may allow a more efficient implementation of an anomaly detection. For each of the workflow phases the current incoming sensor signals may be compared to the expected sensor signals (derived from measurements during normal operation on healthy equipment). The anomaly detection may comprise identifying and/or detecting a deviation from normal signals (i.e. anomaly) and/or detecting a slowly evolving trend. The evolving trend may be detected, when the sensor signals start to deviate from the normal signals. When such a deviation or trend is detected, the method may decide to send a sample of raw data of the obtained signals from the sensor to the cloud for further detailed analysis, while in the normal situation the raw data of the sensor may be deleted.

The method may allow the local processing to be quite limited. The actual detailed analysis can be done in the cloud without sending all the raw data obtained from sensor to the cloud all the time.

### LIST OF REFERENCE SIGNS:

- S100: obtaining signals
- S200: providing a workflow phase determining algorithm
- S300: determining the workflow phase data
- 100,207: device
- 200: system
- 201: medical system
- 202, 203, 204, 205: sensor
- 206: data connection
- 300, 400, 500: signal
- 301: spectrogram
- 302: start up phase
- 303: speedup rotation phase
- 304: steady rotation phase
- 305: X-ray emission phase
- 306: arc phase
- 307: gantry coast down phase
- 401, 501: workflow phase determining algorithm
- 402: selecting algorithm
- 403, 404, 405, 406, 503: evaluation algorithm
- 502: buffering unit
- 504: data connection
- 505: analysing platform
- 506: local side
- 507: external side

## Claims

1. A method for determining a workflow phase of a medical system (201) in a medical environment, comprising:
obtaining, by a processor, signals (300, 400, 500) from at least one sensor (202, 203, 204, 205), wherein the at least one sensor (202, 203, 204, 205) is external to the medical system (201) and configured to monitor the medical system (201) (S100);
providing, by the processor, a workflow phase determining algorithm (401, 501) configured to determine workflow phase data of the medical system (201) (S200);
determining, by the processor, the workflow phase data by utilizing the workflow phase determining algorithm (401, 501) and the obtained signals (300, 400, 500) (S300).

2. The method according to claim 1, comprising:
using, by the processor, the workflow phase data to select at least one evaluation algorithm (403, 404, 405, 406, 503) for a medical system (201) corresponding to a workflow phase comprised in the workflow phase data; and/or
providing, by the processor, the determined workflow phase for further processing, wherein the further processing comprises displaying the workflow phase and/or analysing the workflow phase.

3. The method according to claim 1 or 2, wherein the at least one evaluation algorithm (403, 404, 405, 406, 503) comprises at least one of the following: a failure detection algorithm of at least a part of the medical system (201), a position estimation algorithm of at least a part of the medical system (201), a failure localization algorithm of at least a part of the medical system (201), a failure prediction algorithm of at least a part of the medical system (201).

4. The method according to any of the preceding claims, comprising executing the at least one selected evaluation algorithm (403, 404, 405, 406, 503), and in particular presenting a result of the executed at least one selected evaluation algorithm (403, 404, 405, 406, 503) on a user interface.

5. The method according to any of the preceding claims, comprising
the processor analysing the determined workflow phase data based on corresponding reference workflow phase data to obtain an analysing result, the analysing result particularly comprising identification of an anomaly in the workflow phase data,
the processor transmitting the obtained signals (300, 400, 500) from the at least one sensor (202, 203, 204, 205) to an analysing platform (505), in particular a platform on a cloud system, via a data connection (504) for further analysis based on analysing result or deleting the obtained signals (300, 400, 500) based on the analysing result.

6. The method according to any of the claims 2 to 5, wherein the at least one selected algorithm is executed on a cloud system, wherein the processor and the cloud system are connected by a data connection (504), and wherein in particular the processor is implemented on an edge device.

7. The method according to any of the preceding claims, further comprising signal processing of the obtained signals (300, 400, 500), wherein signal processing comprises signal conditioning, digitization, transformation and in particular feature extraction.

8. The method according to any of the preceding claims, wherein the workflow phase determining algorithm (401, 501) is based on a machine learning algorithm trained to predict the workflow phase data, in particular the workflow phase comprised in the workflow phase data.

9. The method according to any of the preceding claims, wherein the workflow phase determining algorithm (401, 501) is based on a classification algorithm, in particular a classification algorithm configured to determine the workflow phase based on the obtained signals (300, 400, 500) .

10. The method according to any of the preceding claims, wherein the at least one sensor (202, 203, 204, 205) comprises at least one of the following: an acoustic sensor, a vibration sensor, an electromagnetic sensor and a thermal sensor.

11. The method according to any of the preceding claims, wherein medical system (201) comprises one or more parts and wherein the medical system (201) comprises at least one of the following: a CT system, an X-Ray system, a MR system, an ultrasound system, a catheter system, a respiratory system and a patient table.

12. The method according to any of the preceding claims, wherein the obtained signal stems from a source configured to generate a synthetic signal and wherein the source is positioned on the medical system (201).

13. Device for determining a workflow phase of a medical system (201) in a medical environment, the device comprising means configured to carry out the steps of the method according to any of the claims 1 to 12.

14. System comprising a device according claim 13 and a medical system (201), at least one sensor (202, 203, 204, 205) and optionally a user interface.

15. Computer program comprising instructions which, when the program is executed by a computer, cause the computer to carry out the method according to any of the claims 1 to 12 and/or a computer-readable medium comprising instructions which, when executed by a computer, cause the computer to carry out the method according to any of the claims 1 to 12.
